# EUROPEAN PATENT SPECIFICATION

(11) **EP 1 804 875 B1**
(45) Date of publication and mention of the grant of the patent: **16.05.2018**
(21) Application number: 05791240.4
(22) Date of filing: 07.10.2005
(51) Int. Cl.: A61M 16/06

(54) **PATIENT OXYGEN DELIVERY MASK**
SAUERSTOFFABGABEMASKE
MASQUE À OXYGÈNE POUR PATIENT

(30) Priority: 15.10.2004 US 966920; 20.01.2005 US 38816
(43) Date of publication of application: 11.07.2007
(73) Proprietor: Southmedic Incorporated, Barrie, Ontario L4M 5K3 (CA)
(72) Inventor: MCDONALD, Lee, Barrie, ON L4M 7S5 (CA); HAJGATO, JULIUS c/o Southmedic Incorporated, Barrie, Ontario L4M 5K3 (CA)
(74) Representative: Becker Kurig Straus
(86) International application number: PCT/CA2005/001541
(87) International publication number: WO 2006/039788

(56) References cited:
- EP-A1- 0 053 449
- WO-A1-98/29153
- US-A- 2 313 999
- US-A- 3 295 521
- US-A- 4 201 205
- US-A- 5 233 978
- US-A1- 2004 094 160
- US-B1- 6 450 166

## Description

### FIELD OF THE INVENTION

The present invention relates to a novel mask for delivery of gases such as oxygen or oxygen-enriched gas to a patient, and more particularly to a mask which can be used to replace conventional oxygen masks and nasal cannulae oxygen delivery systems.

### BACKGROUND OF THE INVENTION

Conventional oxygen masks comprise tent like structures which are strapped over the nose and mouth of the patient, often using an elastic band or bands behind the patient's ears or head. Oxygen is fed from a supply through a tube into the bottom portion of the mask at the front of the patient. Many problems exist with such masks, including the fact that many patients find them claustrophobic, the mask must be removed for the patient to speak or eat, thereby discontinuing therapy, and the face mask creates irregular and inefficient infusion of oxygen by the patient since exhaled air from the patient is mixed with oxygen in the mask. Oxygen masks can only be used for oxygen flows greater than 4 litres/minute because exhaled gas accumulates in the mask, and, at lower flow rates interferes with delivery of oxygen-enriched air to the patient.

Conventional nasal cannulae oxygen delivery systems employ an oxygen delivery tube with tubular, open ended nasal prongs at the delivery end of the tube for insertion into a patient's nasal passages. The oxygen delivery tube and nasal cannulae are supported in position by a tube wrapped about the patient's ears or head, making the system both difficult to handle and uncomfortable since it applies downward pressure on the patient's ears when the patient is in a seated position. As well, patients often get nose bleeds from the dryness of the oxygen supplied through the nasal cannulae. Patients also get sores on the ears, face and nose due to the direct contact of the oxygen tubing with the skin. Nasal cannulae can only deliver flows of 0.5 to 4 litres/minute.

Of background interest is Ketchedjian, U.S. Patent No. 6,247,470 issued June 19, 2001 which describes and illustrates an oxygen delivery apparatus comprising a headset to which is pivotally attached, for rotation in one plane, a flexible arm carrying tubular members for passing oxygen to a patient's mouth. The apparatus is also provided with a carbon dioxide monitoring system.

McCombs et al., U.S. Patent No. 6,065,473 issued May 23, 2000 describes a somewhat similar apparatus, for non-medical purposes, intended to dispense concentrated oxygen to users, the apparatus comprising an oxygen delivery nozzle attached by an arm extending from a flexible head band, to bathe the user's nose and mouth with oxygen, when in use. Laid-open German Application DE 43 07 754 A1, published April 7, 1994, teaches a system for controlled supply or removal of respiratory air from a user, which system incorporates a mask body held by a rigid air tube over the mouth and/or nose of the user, the air tube being pivotally adjustable in one plane, to enable proper positioning of the mask.

U.S. Patent No. 3,683,907 of Cotabish issued August 15, 1972 describes and illustrates a fresh air respirator, for use for example by miners, which comprises a cup, supported by pivotable arms in front of the face of the user, a stream of air being conducted to the cup to provide fresh air around the user's nose and mouth.

The applicant has developed a number of lightweight oxygen delivery systems for patients, as described for example in U.S. Patents Nos. 6,675,796 issued January 13, 2004, 6,595,207 issued July 22, 2003 and 6,450,166 issued September 17, 2002. Also, applicant's U.S. Design Patent Nos. D449,376 issued August 16, 2003 and D449,883 issued October 30, 2001 illustrate designs for such devices. All of these references feature oxygen diffuser devices, designed to create a turbulent oxygen flow, to be situated during use in front of the nose and mouth of a patient, and being held in that area by means of a mount such as a head band, to which is secured a rigid, but bendable oxygen delivery tube.

Other references of general background interest include U.S. Patent No. 4,282,869 of Zidulka issued August 11, 1981, U.S. Patent No. 4,018,221 of Rennie issued April 19, 1977, U.S. Patent No. 5,687,715 of Landis et al. issued November 18, 1997, U.S. Patent No. 4,465,067 of Koch et al. issued August 4, 1984 and U.S. Patent No. 5,697,363 of Hart issued December 16, 1977, all of which describe and illustrate different types of head mounted apparatus for delivering oxygen or other gases to a patient.

Most of these prior art devices intended for delivery of oxygen to a patient do not provide the ease of usage, both by health care workers and the patient, and reliability against unintended removal or dislodgement from position, as is required to permit widespread use by the health care profession.

It is an object of the present invention to provide a more versatile, reliable and practical system for delivery of oxygen to patients.

EP 0053449 A1, US 2313999 A, and WO 1998029153 A1 disclose further types of head mounted apparatus for delivering oxygen or other gases to a patient.

### SUMMARY OF INVENTION

The present invention is provided by appended claim 1. Beneficial embodiments are provided in the dependent claims. The following disclosure serves a better understanding of the invention. In accordance with the present disclosure there is provided an improved mask for delivery of oxygen to a patient. The mask comprises a body having a peripheral portion, when in use to sit comfortably on a patient's face, a central portion, and bridge portions extending between the central portion and the peripheral portion and integral therewith. The central portion has an inner surface and an outer surface. The inner surface is oriented towards the patient's face, when the mask is in position, and is contoured so as to sit at a location spaced over the patient's nose and mouth. The inner surface of the central portion is provided with a wall circumscribing a base. The wall and base are of generally concave configuration and circumscribe a centrally positioned oxygen delivery aperture which extends through the central portion between the inner surface and the outer surface. The wall and base are configured so as to act as an oxygen diffuser to direct the flow of oxygen generally towards the patient's nose and mouth when the mask is in use. The mask is provided with a fastener to engage the mask to the patient so as to maintain contact between the patient's face and the peripheral portion of the mask when the mask is in use. Also, means are associated with the aperture of the central portion to releasably secure in position an oxygen delivery tube. In a further embodiment of the present disclosure, the mask additionally includes the oxygen delivery tube. It is releasably securable to the outer surface of the central portion of the mask so as to communicate with the oxygen delivery aperture. As well, a baffle is provided, the baffle being constructed so as to be releasably seated over the oxygen delivery aperture on the inner surface of the central portion of the mask. The inner surface of the baffle is configured so as to assist, during use of the mask, in creating turbulence in an oxygen flow leaving the oxygen delivery aperture and assist in mixing oxygen with ambient air and thereby avoid a direct flow of oxygen towards the patient's face.

In a yet a further embodiment of the present disclosure, the mask is further provided with an oxygen/carbon dioxide monitor tube releasably securable to the outer surface of the central portion of the mask, so as to communicate through the oxygen delivery aperture with an area above the inner surface of the central portion during use of the mask for passage of air within the mask to an oxygen/dioxide monitor. The baffle is constructed so as to be releasably seated over the oxygen delivery aperture on the inner surface of the central portion of the mask. The baffle has a concave shaped wall and is configured and positioned so as to assist during use of the mask in creating turbulence in an oxygen flow leaving the oxygen delivery aperture and assist in mixing oxygen with ambient air and thereby avoid a direct flow of oxygen toward a patient's face. A carbon dioxide intake is positioned within the concave shaped wall of the baffle, the carbon dioxide intake communicating with the carbon dioxide monitor tube. The bridge portions of the mask, from a top of the mask to a bottom of the mask, may be configured in an inverted "Y" shape so that openings are provided towards the bottom and on both sides of the mask for unobstructed access to, and viewing of, a patient's mouth and other parts of the patient's face. The bridge portions comprise a web which is spaced apart from the patient's face so as to maintain a sense of openness and lack of confinement for the patient. A suitable spacing is between 12 and 40 mm from the patient's face, for example when measured from the region between the patient's nose and upper lip. In a further aspect, the oxygen delivery system comprises in general terms a mask for covering a portion of a patient's face, having a peripheral rim for contacting the patient's face to substantially surround only his mouth and nose region, a web-like mask body and an oxygen diffuser. A fastener such as an elastic strap holds the mask firmly against the patient's face so as to cover the nose and mouth region. The body is semi-rigid or more preferably rigid and shaped so as to protrude outwardly away from the patient's face and to be spaced apart from the user's face when in use. The body may be provided in a range of sizes to fit different sized patients, such as infants, normal adults and those with larger than average faces. The body supports a gas diffuser and a baffle or other gas obstructing member positioned in the path of the discharged gas so as to direct a turbulent stream of gas towards the user, such as oxygen or an oxygen-rich gas. The diffuser is positioned so as to direct the gas flow towards the user's mouth and nose region at a non-oblique angle so as to generate a plume covering or substantially covering this portion of the patient's face. Thus, if the user is upright the gas flow is substantially horizontal. The mask body consists of a web having at least one and preferably a plurality of openings to permit access to the patient's face, for example an opening directly opposed to the patient's mouth to permit the user to eat, drink and converse normally while wearing the mask, while also permitting medical personnel to easily administer medicines or a thermometer or the like to the patient's mouth. A plurality of relatively large openings also permits rapid discharge and dispersal of exhaled gases to the exterior of the mask. The location, size, number and shape of the openings is dictated at least in part by the desired use of the mask, keeping in mind the comfort and convenience of the patient and the needs of medical staff for potential access to the patient's mouth or nose. For example, a mask for use by an infant may include openings which permit the feeding of the infant, wiping of his face and other functions. Medical staff may also need to have quick access to the patient's nose or mouth, and it is convenient that this may be accomplished without removal of the mask. Another important aspect which dictates the opening size etc. relates to psychological factors, to minimize the anxiety felt by users. This can be particularly the case with infants, the elderly and those suffering from diminished mental capacity. Thus, a variety of different configurations of the openings is contemplated. The mask has a size and shape so as to provide an oxygen-enriched zone surrounding the patient's nose and mouth. The openings within the mask body can take on any convenient and suitable shape such as square, triangular or rectangular or other polygonal, or round or oval. The openings have a size range of between 25 square inches in area and 6.0 square inches and preferably between 0.25 and 3.0 square inches. The size and shape of the mask is preferably optimized to minimize the surface area of the mask body and maximize the open area so as to enhance the user's comfort. This is accomplished by providing a relatively large area of the mask body being open, for example by providing a plurality of relatively large openings within the body. It has been found that the mask body may comprise a relatively open structure if the gas diffuser mounted to the mask is positioned generally directly opposed to the patient's nose and mouth region and spaced apart from the patient's face so as to generate a plume of turbulent gas flow which covers the mouth and nose region of the patient. In order to generate this plume of turbulent flow, the diffuser comprises a rear wall which fixedly receives a gas delivery tube, the mouth or nozzle of the tube discharging into the interior of the diffuser towards the patient's face. A gas flow disrupter is positioned between the nozzle and the patient's face. The rear portion is surrounded by a peripheral wall or flange extending towards the interior of the mask and the patient's face so as to form a generally concave structure to assist in directing the gas flow towards the user. Preferably the rear portion is a wall which may be flat or curved, with the gas outlet entering the wall, for example at a generally central location. The peripheral wall may comprise a generally triangular shape as in the above-described embodiment, or any other convenient shape. A turbulent flow pattern is generated by providing one or more obstructions associated with the diffuser in the path of the gas flow after it exits the gas delivery tube. For example, a baffle may be provided within the interior of the diffuser, which may comprise a mushroom-shaped structure which partly obstructs the path of the discharged gas. Alternatively to a gas flow disrupter other means may provide a turbulent gas flow, for example the shaping of the nozzle itself.

The diffuser preferably conforms to dimensional and positional constraints described herein in order to provide a gas plume having sufficient size to substantially cover the user's nose and mouth. It has been found that the diffuser should comprise a width of no more than 40 mm at its widest point and a maximum height of 80 mm and preferably smaller than this in both dimensions. The diffuser is positioned within the mask such that no part of the diffuser is outside of a region defined in relation to a point between the base of the user's nose and his upper lip, the region comprising a space 40 mm above and below this point in the vertical plane when the mask is vertical and 20 mm on either side of this point in the side- to-side direction. Preferably the diffuser is centered in side to side and vertical dimensions relative to this point. Further, the diffuser is preferably mounted so as to leave a gap between the diffuser and the user's face of between 12 and 40 mm as measured from the user's skin surface between upper lip and nose. Preferably, the diffuser is generally triangular in shape with base downwardly, and preferably the width and height are between 20- 30 mm and 17-27 mm respectively. It has been found that a mask which is provided with a diffuser that conforms to the dimensional requirements described above, and which is positioned within the mask as described, and wherein the mask body includes a plurality of openings or cut-outs, provides an optimal level of comfort, with minimal coverage of the face while still permitting an oxygen- enriched zone fully covering the user's nose and mouth. Preferably the cut- outs within the mask body should comprise at least 30 per cent of the total surface area of the body when measured as if the body were considered to comprise a plane surface, and still more preferably the cut-outs comprise between 30 and 80 per cent of the total surface area. In a still more preferred version the range is more narrowly defined as being between 50 and 80 per cent, and yet more narrowly as between 60 and 75 per cent. An oxygen delivery mask permits an efficient delivery of oxygen- enriched gas in a comfortable fashion if it includes a combination of an oxygen diffuser opposed to the user's nose and mouth region and directed so as to direct a turbulent gas plume directly at the nose and mouth, and at least one opening within the mask body. Without intending to be restricted to a theory, it is believed that the user's breath inspiration generates negative pressure within the mask interior thereby creating a mixing effect whereby exterior air is drawn through the mask openings into the turbulent gas plume via a venturi effect. Upon exhaling, a positive pressure is generated within the mask and the exhaled air is exhausted through the openings. This maintains within the mask interior an oxygen-rich and CO2- poor environment, which enhances the user's comfort level and is more medically effective.

Positioning of the diffuser opposed to the nose and mouth region so as to direct a turbulent plume directly towards the user's nose and mouth also permits an oxygen-enriched gas plume which is of generally equal gas makeup over both the mouth and nose. Thus, user inhales the same gas mixture whether breathing through mouth or nose. The diffuser is suitable shaped and positioned so as to cover both the nose and mouth of a typical user.

The oxygen delivery mask of the present disclosure provides an easy to use, comfortable, reliable and efficient mask for delivery of oxygen to a patient. As well, since this mask construction does not provide complete enclosure over the patient's nose and mouth, there is less chance of claustrophobia.

In a further aspect, although the disclosure has been described for use for delivering oxygen or an oxygen-enriched gas it will be seen that with modifications it may be used for other medical applications such as delivery of anesthetic or other gases to a patient.

### BRIEF DESCRIPTION OF THE DRAWINGS

These and other advantages of the disclosure will become apparent upon reading the following detailed description and upon referring to the drawings in which:
FIGURE 1 is an exploded perspective view from the rear of one embodiment of the oxygen delivery mask according to the present disclosure;
FIGURE 2 is an elevational section view of the mask of Figure 1, along lines 2-2 of Figure 1;
FIGURE 3 is a perspective view from the front of the mask of Figure 1;
FIGURE 4 is an elevational section view of an alternative embodiment of oxygen delivery mask in accordance with the present disclosure, including a carbon dioxide monitoring function; and
FIGURE 5 is a front elevation view of the mask of Figure 1.
FIGURES 6A, 6B and 6C are front elevational views of further alternative embodiments of the mask.
FIGURE 7 is a perspective view of a further embodiment of the mask; FIGURE 8 is a further perspective view of the embodiment of Figure 7.
FIGURE 9 is a side elevational view of the embodiment of Figure 7.

While the disclosure will be described in conjunction with illustrated embodiments, it will be understood that it is not intended to limit the disclosure to such embodiments. On the contrary, it is intended to cover all alternatives, modifications and equivalents as may be included within scope of the disclosure as defined by the specification as a whole. It will be understood that dimensions and relative dimensions described and illustrated herein are intended to be by way of example only of specific embodiments and unless otherwise indicated are not intended to limit the scope of the disclosure. References herein within both the description and claims to specific directions and positions such as "horizontal", "vertical", "forward" and the like are intended only to provide a convenient means of description and are intended to be in reference to the mask in an upright forward -facing position, as if it were worn by a patient in a standing position. Naturally the mask may be used on a patient in any orientation.

### DETAILED DESCRIPTION OF THE PREFERRED EMBODIMENTS

In the following description similar features in the drawings have been given similar reference numerals.

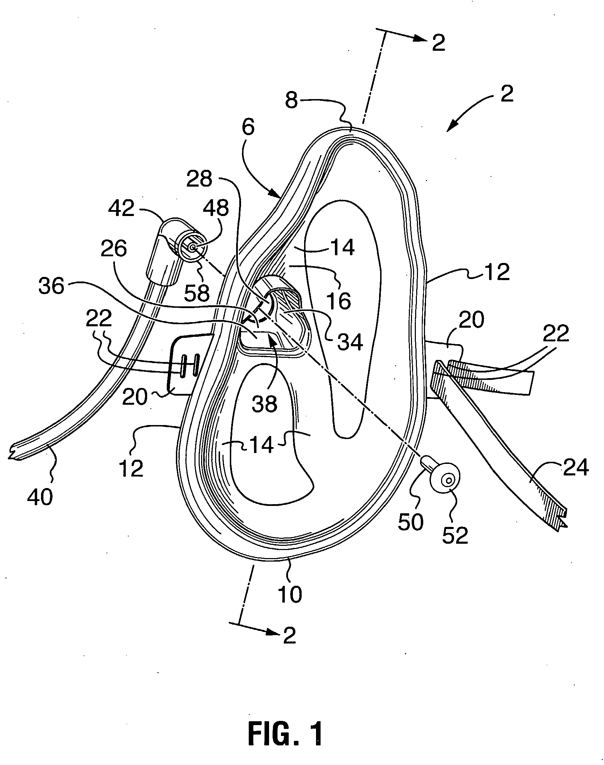

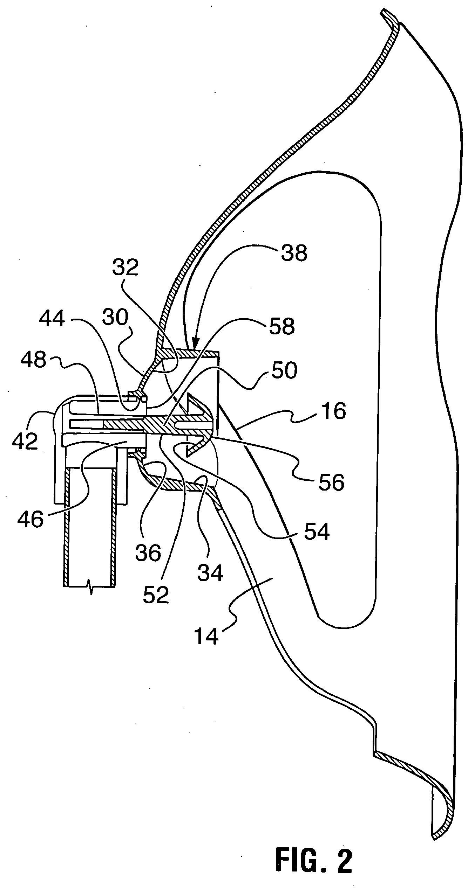

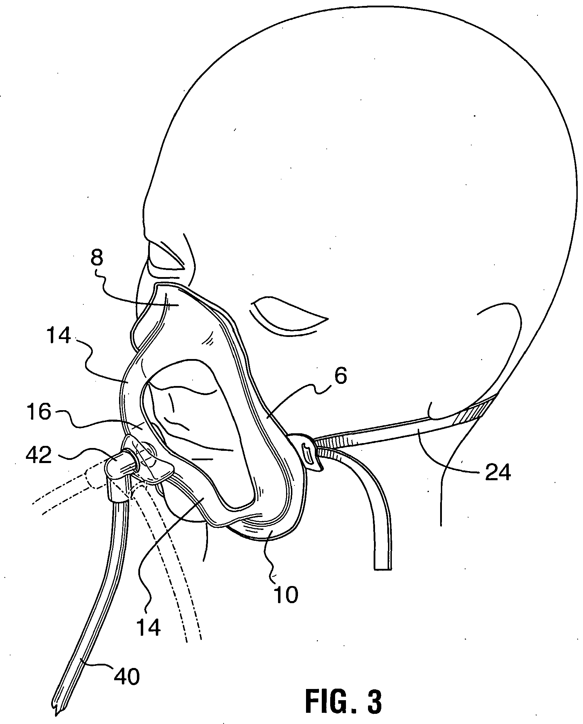

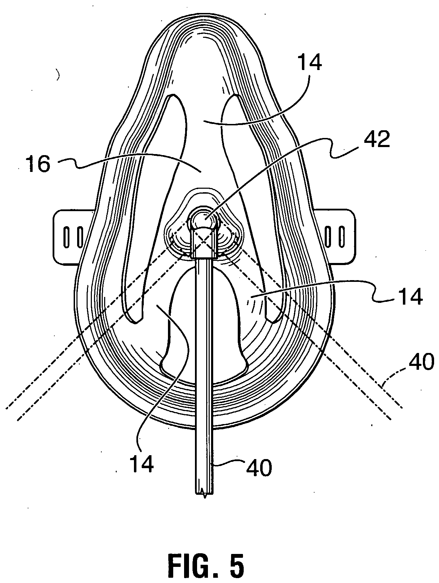

Turning to Figures 1 and 2 there is illustrated an oxygen delivery mask 2 which represents an embodiment of the present invention. Mask 2 is made up of a body 4 having a peripheral portion 6 with a top 8 and a bottom 10. Sides 12 extend between top 8 and bottom 10. As can be seen in Figure 3, peripheral portion 6, when mask 2 is in use, rests on portions of a user's face both above the user's nose (top 8) and on the user's chin (bottom 10). Integrally formed with peripheral portion 6 are bridge portions 14 which integrally connect with a central portion 16. Bridge portions 14 and central portion 16 have an inverted "Y" shaped configuration (from top to bottom of the mask), when viewed from the front (Figure 5), providing unobstructed access to and viewing of the patient's mouth and other parts of the patient's face, so that for example, the patient may eat and drink without removing the mask. Of course other configurations of bridge portions may be provided as desired or appropriate, such as, for example, an "X" shape, a "+" shape or "T" shape. Peripheral portion 6, bridge portions 14 and central portion 16 are preferably made of a fairly soft, semi rigid plastic material. The term "semi-rigid" refers to a material that is generally resilient but which provides sufficient rigidity to substantially maintain its shape when in normal use. The bridge portions 14 thus have sufficient rigidity to retain the diffuser 38, discussed below, in position. Suitable materials for the peripheral, bridge and central portions 6, 14 and 16 include plastics such as PVC, silicone, foam, polystyrene and any other thermoplastic elastomers. Tabs 20 extend outwardly from sides 12, and are provided with, for example, slots 22 in which may be adjustably secured ends of an elastic strap 24 for releasably securing the mask 2 in position on a user's face (Figure 3). As will be understood from Figures 1 and 3, peripheral portion 6, bridge portions 14 and central portion 16 are contoured so as to rise from base 26 of central portion 16 in a curved contour so that central portion 16 sits spaced over the nose and mouth of the patient when the mask 2 is in position. A circular aperture 28 extends through central portion 16 from outer surface 30 to inner surface 32.

Integrally formed on inner surface 32 of central portion 16, is a triangular wall 34, extending about a base 36 which circumscribes circular aperture 28. This wall 34 and base 36 together form a shape which is of generally concave configuration, with one of the apexes of the triangle formed by wall 34 being oriented towards top 8 of mask 2 and the other two apexes oriented towards bottom 10. Thus, a generally triangular shape is formed by the mask, albeit generally with rounded corners, which approximates the human nose and mouth region, so as to generate an oxygen enriched region within the mask which substantially covers the user's nose and mouth. This wall and base form a diffuser 38 which has a similar function to the diffuser construction described and illustrated in applicant's earlier patents and applications referred to previously herein. The diffuser 38 preferably has a maximum width of about 40 mm, with a more preferred maximum width of 30 mm and still more preferred maximum being 25 mm. A maximum height is preferred to be 80 mm, with increasing preferences being 27 mm and about 22 mm.

In the embodiment of mask illustrated in Figures 1 to 3, an oxygen delivery tube 40 is secured to a connection in the form of a rigid elbow 42, elbow 42 being rotatably secured by an appropriate, conventional securing means such as frictional engagement in aperture 28 or its snapping into an undercut 44 about aperture 28 on the outer surface 30, so that it can pivot about the circumference of aperture 28 (Figures 3 and 5). Elbow 42 provides a passageway 46 for delivery of oxygen, during operation of the device, into diffuser 38 on the inner surface 32 of central portion 16. The diffuser 38 and associated baffle are shaped so as to generate a plume of turbulent flow of oxygen-enriched gas which surrounds the user's nose and preferably also his mouth. The generation of this plume requires a suitable discharge velocity of gas through the nozzle. This may be accomplished by providing a gas discharge rate between 1- 15 liters/min. Formation of the plume is related to the physical design of the nozzle, diffuser and baffle. The blending of the oxygen and atmospheric oxygen is related to the gas velocity. Without wishing to be tied to any particular theory of operation, the physical properties of the device required to generate a plume include the shape, size and positioning of the baffle which serves to deflect the gas flow back down, then bounce off sidewalls to create this plume. The direction of the gas flow exiting the diffuser is substantially directly towards the user's face so as to strike the user at an angle generally perpendicular to the user's face. The gas flow is thus non-oblique in relation to the user's face. The diffuser 38 is positioned so as to direct the flow most strongly at the user's nose and mouth region. It has been found that the configuration described herein efficiently provides an oxygen-enriched zone in the region surrounding the user's mouth and nose, while permitting relatively large cut-outs within the mask.

The baffle 52 is positioned within the path of the gas exiting the nozzle and has a shape and size which is suitable for interrupting the linear gas flow exiting the nozzle so as to generate turbulence. It will be seen that a variety of sizes and shapes will achieve this function. In the illustrated example, the baffle comprises an upstanding stem 48 within elbow 42 which provides a means for releasable attachment thereto of post 50 of mushroom shaped baffle 52. As can be seen in Figure 2, the inner end of baffle 52 has a curled back conical lip 54 on its head 56, the underside of this lip being in line with oxygen passing from aperture 28 at the inner end 58 of elbow 42. This curled back conical lip 54 is of a size and configuration, with respect to wall 34 of diffuser 38, such that turbulence is generated in the stream of oxygen passing from elbow inner end 58 and aperture 28, creating a plume of oxygen enriched air at the patient's nose and mouth when the mask is in position.

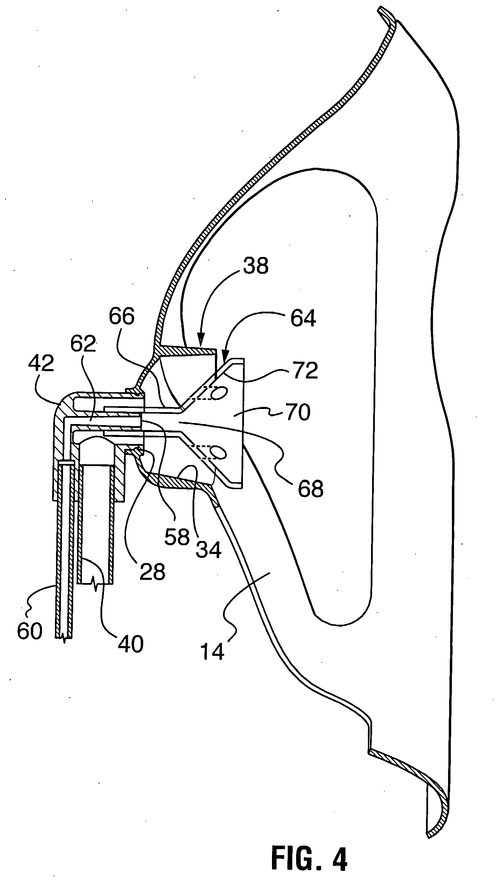

In the alternative embodiment of mask 2 illustrated in Figure 4, while mask body 4 and integral diffuser 38 are of a similar configuration to those of Figures 1, 2 and 3, in addition to an oxygen delivery tube 40 passing into elbow 42, elbow 42 is configured to have an oxygen/carbon dioxide monitor tube 60 secured to it, which tube communicates with a separate oxygen/carbon dioxide monitor passageway 62 extending within elbow 42 to its inner end 58. Oxygen/carbon dioxide monitor tube 60 and passageway 62 are separate and independent from oxygen delivery tube 40 and oxygen delivery passageway 46. Oxygen from delivery tube 40 is again delivered through elbow 42 to aperture 28 and the inside of mask 2 and the wall 34 of diffuser 38 circumscribes this aperture 28 and directs the flow of oxygen generally outwardly from diffuser 38.

In this embodiment, baffle 64 has a hollow post 66, the hollow center communicating with an opening 68 on the inside of baffle 64, and with the oxygen/carbon dioxide monitor passageway 62 and tube 60.

Head 70 of the baffle 64 circumscribes the opening 68, the head being of a concave shape formed by wall 72. This head 70 fills a significant part of the interior of diffuser 38. Wall 72 extends outwardly beyond the edges of wall 34, and generates the necessary oxygen turbulence to provide an effective plume of oxygen for delivery to the nose and mouth area of the patient when the mask 2 is in position. At the same time however, an effective oxygen/carbon dioxide monitoring of the patient's exhaled breath is permitted through the oxygen/carbon dioxide monitor opening 68 within head 70.

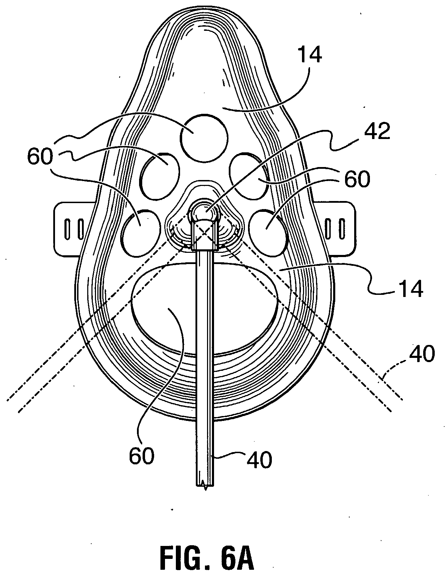

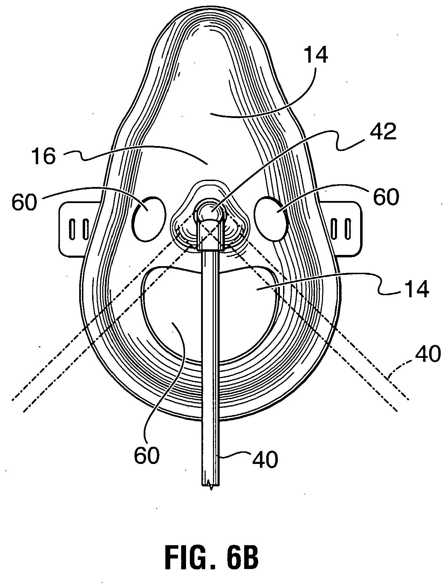

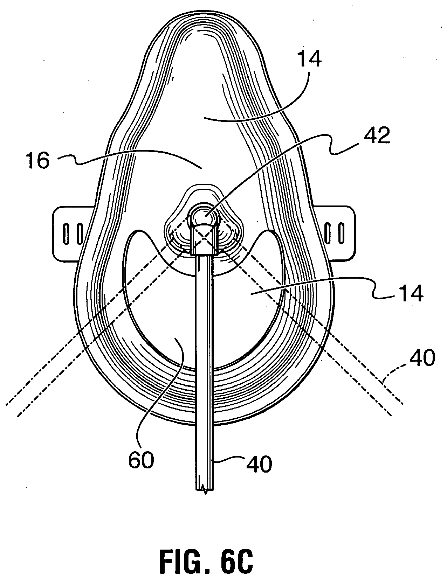

In further embodiments shown in Figures 6A, 6B and 6C, a mask 2 comprises a body 4 having a peripheral rim 6. The rim 6 comprises a material such as PVC or silicone which provides sufficient rigidity to permit the mask to maintain its shape while permitting a degree of resiliency to permit the mask to comfortably conform to a user's face. The rim is provided with an outer surface for contacting the patient's face of a material which is soft and pliable plastic with a Durometer range of 20-100 Shore A. The rim preferably has a roughly triangular shape to generally conform to a human nose and mouth region, namely a broad base and a narrow apex, with rounded corners. The rim 6 may alternatively comprise a bendable material such as aluminum which retains its shape when flexed, which may be provided to the user in a shape which roughly follows the contours of a typical human face. The mask may be provided in a plurality of rim sizes to fit different classes of users, for example infants and small, medium and large adults.

The body 4 comprises a web of shaped substantially rigid material. This may comprise a semi-rigid material such as that described above in connection with the first embodiment. However, if the body 4 is provided with a more lace-like structure defined by a large number of cut-outs, described below, a more rigid material will be desired such as TPE-"thermoplastic elastomer". In general, the body has sufficient rigidity to maintain its generally cup-like configuration and to support the diffuser 38 which is fastened to and supported by the body, at a position spaced apart from the user's face. Because the relative position of the diffuser 38 is important to the functioning of this version, the body should have sufficient rigidity to maintain the central position of the diffuser 38 during normal use of the mask. The body 4 may possess a degree of resiliency to enhance user comfort and daily functions and to permit the mask to better conform to the user's face. The body comprises a plurality of cut-outs 60 which may be of any shape and size suitable to serve several desired functions. These functions include permitting the user to speak, eat and drink with a minimum of obstruction, wipe or blow his nose, scratch or otherwise touch his face, kiss and other normal activities. As well, it is contemplated that others such as health professionals may rely on the cut-outs 60 to feed or provide fluid to the user or for other functions. Thus, it is contemplated that relatively large openings are provided in the region of the patient's nose and mouth. However, other arrangements of openings are possible such a larger number of smaller openings. The openings must not be so many or large as limit the ability of the web to fixedly support the diffuser 38.

The diffuser 38 is mounted within the mask body 4 so as to be centered vertically and horizontally (side-to-side) above a point which when the mask is worn by a person, is about halfway between the base of the nose and the upper lip.

Preferably, the cut-outs 60 comprise at least 50 per cent of the total surface area of the body 6 (when measured with the surface area including both open and closed areas) and still more preferably the cut-outs comprise between 50 and 80 per cent of the total surface area. In a still more preferred version the range is more narrowly defined as being between 60 and 75 per cent.

The diffuser 38 protrudes through and is supported by the body 4 at a position generally opposed to the patient's nose and mouth region. The diffuser and associated baffle are substantially as described in connection with the embodiments. However, it has been found that if the diffuser body conforms to particular size and positioning limits, it effectively generates a zone or region of oxygen-enriched gas in the region of the patient's nose and mouth, regardless of the number, size and shape of the cut-outs 60. For this purpose it is desirable to fabricate the body 4 from a relatively rigid material such as polystyrene, thermoplastic elastomer or polycarbonate. The diffuser 38 receives a gas supply from supply line 40, connected via elbow 42 in the same manner as described above. A mushroom-shaped baffle within the interior of the diffuser 38 assists in the dispersal of gas. It will be further apparent to those skilled in the art that the diffuser 38 may comprise a range of sizes and shapes. However, in order to generate the desired region of oxygen enriched gas the diffuser comprises a cup-like body opening towards the user. It should have a maximum size and positioning within the mask that permits the diffuser to fit entirely within a region of the mask defined by reference to an imaginary point on the user's face between the base of the nose and upper lip, in the midline of the face, with the diffuser entirely fitting within the space defined by 20 mm on either side of this line horizontally and 40 mm above and below the this point vertically (when the user is upright). Preferably the diffuser is sufficiently small so as to permit some slippage of the mask while still remaining within this region, for example as described above a generally triangular configuration about 25 mm wide at its base and about 22 mm high. The diffuser is also positioned within the mask such that the gas discharge nozzle is between 12 and 40 mm displaced forwardly of the user's face measured from the area between the upper lip and below the nose of a users face. The body 6 must have sufficient forward protrusion so as to position the rear rim of the diffuser so that it does not contact the nose of the typical user. The spacing thus required will vary somewhat with different sizes of masks. For example, the diffuser may protrude rearwardly into the interior of the body 4 by about 2 mm, and the body thus has an overall depth of about 15 mm.

As well, the mask design of the present invention allows a patient to drink, eat, be suctioned and speak, without removal of the mask. Also, exhaled air does not collect in the area in front of the patient's nose and mouth and interfere with the mask's operation, as in the case of conventional oxygen masks, since exhaled air easily passes to the surrounding environment through the spaces between the bridge portions and the peripheral portion of the mask.

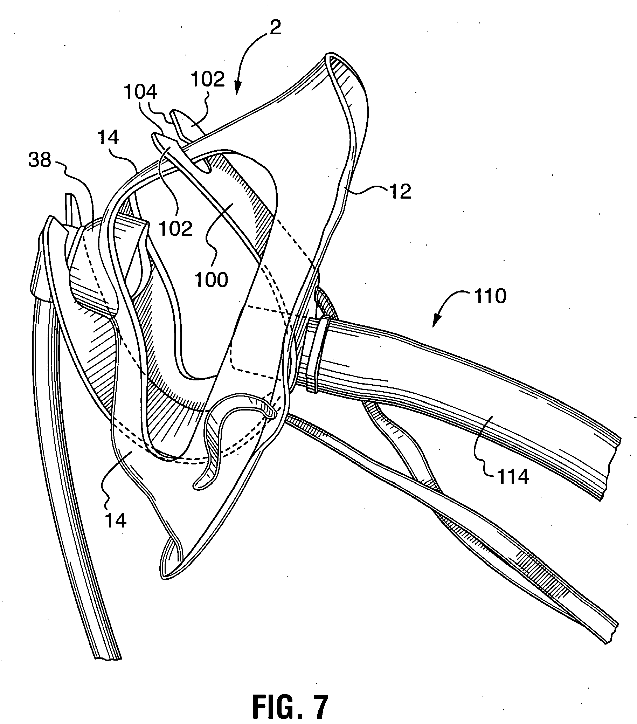

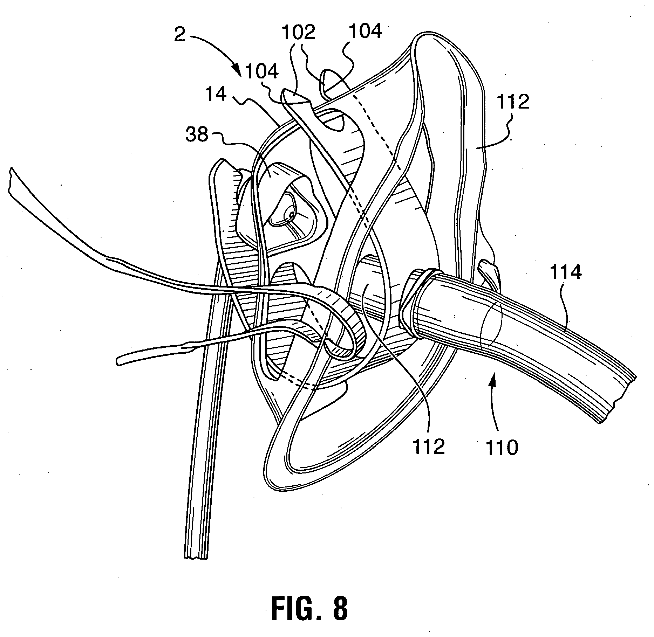

A further embodiment of the mask is illustrated at Figures 7, 8, and 9, wherein the oxygen delivery mask 2 is provided with a removable packing support and retainer strap 100. The strap 100 is made from a semi-rigid material such as polypropylene or other plastic, and comprises a generally elongate body having opposing yoke-shaped ends defined by spaced apart arms 102. The arms 102 each have inside edges including inwardly-extending projections 104. The projections 104 are spaced apart to permit a portion of the mask to be inserted past the projections 104, into a partially enclosed space 105 within the yoke-shaped end regions which thereby retains the strap to the mask. The end regions 102 are intended to releasably receive spaced apart portions of the mask that conveniently fit within the interior space 105 and are retained therein by the projections 104. For example, the opposed ends may respectively receive the bridge portion 14 and the elbow 42 of the mask 2. It will be seen that the strap 100 must be bent into a substantially U-shaped form for this purpose. The strap 100 when flexed into this position is useful during shipping and storage of the mask to help maintain its proper shape and avoid compression or crushing. The strap 100 may be removed prior to conventional use of the mask. However, the strap 100 is also intended to serve a second function and for this purpose it is left in place on the mask, as will be described below.

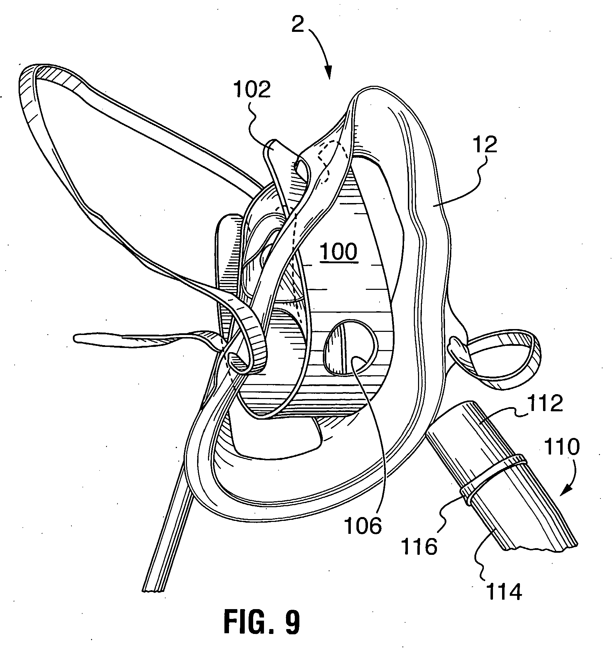

The strap 100 includes a circular opening 106, located between the opposing ends 102. The opening 106 has a suitable dimension to snuggly receive an end of a laryngeal mask 110. The laryngeal mask is a tube-shaped article intended to be inserted down the throat of a patient who has an obstruction in the throat, thereby assisting in the breathing for such patient. The laryngeal mask (shown in part in the figures) includes a first end to receive oxygen from the oxygen diffuser. The first end includes a rigid tubular insert 112, which is fitted within a flexible tube 114. The insert 112 includes an outwardly-protruding flange 116. The insert 112 fits snuggly within the opening 106, up to the limit defined by flange 116, which serves as an effective stopper for the tube. The snug fit of the insert 112 provides a friction fit within the strap 100. As will be seen in Figure 9, the open end of insert 112 is spaced apart from and opens towards the diffuser 38, so as to receive gas from within the plume of gas generated by the diffuser 38, when in use..

The strap 100 thus serves two functions, the first being as a packing support member to prevent crushing or damage to the mask, for example during transportation, the second purpose being to retain a laryngeal mask, for use by patients in need of the same. Optionally, the strap 100 may serve either or both of these functions.

It will be seen that the strap 100 may be made of any suitable rigid or semi-rigid material, and polypropylene is described herein merely as an example of a suitable material. Similarly, the precise dimensions and shape of the strap 100 are subject to reasonably wide variation. For convenience, the strap is somewhat tapered so as to have a wider middle region and narrower ends. This permits the strap to be easily fastened to the mask, at either end of the strap, while permitting the opening 106 within the middle region of the strap with sufficient material on either side of the opening to prevent tearing or cracking of the strap, in use.

In tests which have been done and proven efficacy of the mask designs according to the present invention, it has been determined that patients find the mask according to the present invention to be far more comfortable than conventional oxygen masks. Unlike conventional masks, users cannot feel oxygen being delivered to their nose and mouth area, and enjoy the compactness of the mask. Technically, lower flow rates of oxygen to a patient through the mask of the present invention can be achieved, with as much or greater oxygen concentration in the air being delivered to the patient, as compared to conventional oxygen masks. In this manner, the mask according to the present invention provides both comfort and efficiency to patients which providing optimal blood oxygen saturation in a cost effective manner. Flow rates ranging from .5 liters to 15 liters per minute have proven suitable providing a far greater range of possible flow rates than available through conventional oxygen delivery devices. The following examples describe tests performed with the mask.

### Example 1

A study was conducted, comprising a randomized, cross-over, single-blind study of patients having the following inclusion criteria:
- chronic pulmonary disease requiring supplemental oxygen therapy;
- stable oxygen requirement (unchanging over a three hour period);
- 18-80 years of age.

Excluded were patients whose oxygen requirement was unstable (i.e. changing hourly) or who could not tolerate being deprived of supplemental oxygen for five minutes or less.

### Protocol

Continuous monitoring of minute ventilation (almost non-traceable oxygen movement) by Respitrace™, SaO₂ (% oxygen saturation of hemoglobin), HR, nasal/oral flow, PO₂ (mmHg of oxygen in arterial blood) and PCO₂ (% CO₂) at the lip, O₂ flow and TcPCO₂:
- 5-10 min washout (point when the waveform tracing begins to have a flatter peak) / 5 min baseline (room air);
- Mask #1 (supplemental oxygen; referred to herein as "OxyMask™ or "OM" and comprising an embodiment of the invention);
   - 15-30 min at 4-5% above baseline SaO₂;
   - 15-30 min at 8-9% above baseline SaO₂;
- 5-10 min washout / 5 min baseline (room air);
- Mask #2 (supplemental oxygen; referred to herein as "venturi" or "V", comprising a prior art mask);
   - 15-50 min at 4-5% above baseline SaO₂;
   - 15-30 min at 8-9% above baseline SaO₂.

Data analyzed using two-way analysis of variance (ANOVA) and paired t-test:
- p values <0.05 were considered statistically significant.

### Patient Demographics

N=13 patients with chronic pulmonary disease using supplemental oxygen via nasal cannula.
4 male, 9 female.
age: 56 ± 16 (range: 28-79).
BMI: 35.0 ± 12.3.
02 requirement: 2.3 ± 1.3 Lpm (rest), 3.4 ± 1.6 Lpm (exercise).

### Pulmonary Function Tests

| **Spirometry** | | |
|---|---|---|
| | Measured | % Predicted (i.e. as compared to normal values) |
| FVC (forced vital capacity), I | 1.87 ± 0.66 | 57.38 ± 13.20 |
| FEV1 (forced expiratory volume in 1 second), I | 1.22 ± 0.56 | 51.54 ± 21.50 |
| FEV1/FVC | 65.06 ± 19.29 | - |
| V50 (flow at 50% expired FVC), I/sec | 1.38 ± 1.23 | 42.15 ± 34.71 |
| V25 (flow at the last 25% of expired FVC), I/sec | 0.40 ± 0.32 | 25.23 ± 18.66 |
| VC (vital capacity, not forced), I | 2.00 ± 0.80 | 60.20 ± 15.05 |

| **Arterial Blood Gases** | | |
|---|---|---|
| pH | 7.38 ± 0.05 | |
| H ion, nmol/l | 41.63 ± 5.01 | |
| pCO2, mmHg | 47.50 ± 6.65 | |
| pO2, mmHg | 50.88 ± 7.92 | |
| Bicarbonate, mmol/l | 29.13 ± 4.70 | |
| Measured 02 Saturation | 0.85 ± 0.06 | |
| Base Excess, mmol/l | 2.26 ± 4.29 | |

### Results

| | **Low Saturation** | | **High Saturation** | | **ANOVA** | | |
|---|---|---|---|---|---|---|---|
| | OxyMask | Venturi | OxyMask | Venturi | Saturation Level | Mask | Interaction |
| SaO₂, % | 92.0 ± 3.6 | 91.7 ± 3.4 | 94.8 ± 3.2 | 94.9 ± 3.6 | - | NS | NS |
| Flow O₂, L/min | 0.9 ± 0.3 | 4.8 ± 1.5 | 2.1 ± 0.9 | 12.2 ± 3.9 | <0.01 | <0.01 | <0.01 |
| Ve, L/min | 9.1 ± 5.0 | 7.4 ± 4.2 | 10.6 ± 5.9 | 8.0 ± 4.4 | NS | <0.05 | NS |
| tPCO₂, mmHg | 51.9 ± 8.9 | 51.4 ± 7.6 | 51.3 ± 9.1 | 52.4 ± 8.0 | NS | NS | <0.05 |
| P_{I}O₂, mmHg | 229.7 ± 44.5 | 192.6 ± 11.9 | 459.5 ± 167.5 | 330.0 ± 126.6 | <0.01 | <0.01 | <0.05 |
| P_{E}O₂, mmHg | 164.4 ± 16.9 | 181.7 ± 12.2 | 209.2 ± 39.2 | 266.9 ± 52.4 | <0.05 | <0.01 | <0.05 |
| P_{I}CO₂, mmHg | 3.9 ± 1.5 | 1.6 ± 0.9 | 3.2 ± 0.6 | 1.4 ± 0.8 | <0.05 | < 0.01 | NS |
| P_{E}CO₂, mmHg | 33.5 ± 8.9 | 11.3 ± 5.6 | 27.2 ± 8.9 | 11.6 ± 8.0 | <0.01 | <0.01 | <0.05 |
| HR, b/min | 77.9 ± 18.0 | 78.2 ± 17.9 | 78.9 ± 18.9 | 77.3 ± 17.7 | <0.05 | NS | NS |
| Nasal:Oral Flow | 1.20 ± 0.32 | 1.01 ± 0.26 | 1.11 ± 0.22 | 1.07 ± 0.26 | NS | NS | NS |

### Summary

O₂ flow rate significantly lower with OM vs. V.

P_{I}O₂ significantly higher and P_{E}O₂ significantly lower with OM. vs. V

Ve significantly higher with OM vs. V while TcPCO₂ similar between masks.

PiCO₂ and P_{E}CO₂ significantly higher with OM vs. V.

Difference in O₂ flow remained significant when comparing patients whose Ve increased by >=10% with OM vs. V to those whose did not.

It is evident that many alternatives, modifications and variations will be apparent to those skilled in the art in light of the foregoing description. Accordingly, it is intended to embrace all such alternatives, modifications and variations as fall within the scope of the invention as defined by the appended claims.

## Claims

1. A mask (2) for delivery of oxygen to a patient, the mask comprising:
a body (4) having a peripheral portion (6), when in use to sit comfortably on a patient's face, a central portion (16), and bridge portions (14) extending between the central portion and the peripheral portion and integral therewith, the central portion (16) having an inner surface (32) and an outer surface (30), the inner surface to be oriented towards the patient's face when the mask is in position and contoured so as to sit at a location spaced over the patient's nose and mouth, the inner surface of the central portion provided with a wall (34) circumscribing a base (36), the wall and base being of generally concave configuration and circumscribing a centrally positioned oxygen delivery aperture (28) extending through the central portion between the inner surface and the outer surface, the wall and base configured so as to act as an oxygen diffuser (38) to direct the flow of oxygen generally towards the patient's nose and mouth when the mask is in use;
a fastener (24) to engage the mask to the patient so as to maintain contact between the patient's face and said peripheral portion when the mask is in use;
means (44) associated with the aperture (28) of the central portion (16) to releasably receive and secure in position an oxygen delivery tube (40); said diffuser (38) being positioned within the mask; and
abaffle (52, 64) associated with said diffuser in the path of the flow of oxygen;
wherein the diffuser wall (34) and base (36) are of cup-shaped appearance, the wall ending in an edge of triangular peripheral contour;
wherein the diffuser and baffle are shaped so as to generate a plume of turbulent flow of oxygen-enriched gas which surrounds the user's nose any preferably also his mouth, when a gas discharge rate of 1-15 liters/minute is provided;
and wherein the mask body (4) comprises a substantially open structure having openings (60) which together comprise between 30 and 80% of the total surface area of the mask body.

2. A mask according to claim 1 in combination with an oxygen delivery tube (40) releasably securable to the outer surface (30) of the central portion (16) of the mask so as to communicate with the oxygen delivery aperture (28).

3. A mask according to claim 1 wherein the bridge portions (14) of the mask from a top of the mask to a bottom of the mask are configured in an inverted "'Y" shape so that openings (60) are provided towards the bottom and sides of the mask for unobstructed access to and viewing of a patient's mouth and other parts of the patient's face.

4. A mask as defined in claim 1, wherein said diffuser (38) is positioned so as to be substantially centered in the side to side direction relative to said patient's face when the mask is worn by the patient.

5. A mask as defined in claim 1, wherein said openings (60) comprise between 60 and 75 per cent of the total surface area of said mask body (4).

6. A mask as defined in claim 1, wherein at least one of said openings (60) provides access from the exterior of the mask to the patient's mouth or nostrils when the mask is worn by a patient.

7. The mask of any one of claims 1 to 6, wherein the diffuser (38) has a maximum width of about 40 mm and a maximum height of about 80 mm.

8. The mask of any one of claims 1 to 7, wherein said baffle (52) comprises a post (50) and a head (56) having a curled back conical lip (54), the underside of the lip being in-line with oxygen passing from the aperture (28).

9. The mask of any one of claims 1 to 7, wherein said mask further comprises an oxygen or carbon dioxide monitor tube (60) for oxygen or carbon dioxide monitoring.

10. The mask of claim 9, wherein said baffle (64) has a hollow post (66), the hollow centre of which communicates with an opening (68) on the inside of the baffle (64) and with the monitoring tube (60).

## Patentansprüche

1. Maske (2) zur Versorgung eines Patienten mit Sauerstoff, wobei die Maske umfasst:
einen Körper (4) mit einem Randabschnitt (6), um, wenn in Gebrauch, bequem auf dem Gesicht eines Patienten zu sitzen, einen mittleren Abschnitt (16) und Brückenabschnitte (14), die sich zwischen dem mittleren Abschnitt und dem Randabschnitt und integral damit erstrecken, wobei der mittlere Abschnitt (16) eine innere Oberfläche (32) und eine äußere Oberfläche (34) aufweist, wobei die innere Oberfläche in Richtung des Gesichts des Patienten orientiert ist, wenn die Maske in Position ist und so umrissen ist, dass sie an einer Stelle sitzt, die oberhalb der Nase und des Mundes des Patienten beabstandet ist, wobei die innere Oberfläche des mittleren Abschnitts mit einer Wand (34) ausgestattet ist, die eine Basis (36) umgibt, wobei die Wand und Basis eine allgemein konkave Konfiguration aufweisen und eine zentral positionierte Sauerstoffversorgungsöffnung (28) umgeben, die sich durch den mittleren Abschnitt zwischen der inneren Oberfläche und der äußeren Oberfläche erstreckt, wobei die Wand und Basis so konfiguriert sind, dass sie als Sauerstoffdiffusor (38) fungieren, um den Fluss von Sauerstoff in die allgemeine Richtung der Nase und des Mundes des Patienten zu lenken, wenn die Maske in Gebrauch ist;
eine Befestigung (24), um die Maske an dem Patienten anzubringen, so dass Kontakt zwischen dem Gesicht des Patienten und dem Randabschnitt aufrechterhalten wird, wenn die Maske in Gebrauch ist;
Mittel (44), die mit der Öffnung (28) des mittleren Abschnitts (16) assoziiert sind, um einen Sauerstoffversorgungsschlauch (40) lösbar aufzunehmen und in Position zu sichern; wobei der Diffusor (38) in der Maske positioniert ist; und
eine Leitplatte (52, 64), die mit dem Diffusor in dem Weg des Sauerstoffflusses assoziiert ist;
wobei die Diffusorwand (34) und Basis (36) eine napfförmige Erscheinung aufweisen, wobei die Wand in einer Kante mit einem dreieckigen Randumriss endet;
wobei der Diffusor und die Leitplatte so geformt sind, dass sie eine Gasfahne eines turbulenten Flusses eines mit Sauerstoff angereicherten Gases erzeugen, die die Nase des Benutzers und bevorzugt auch seinen Mund umgibt, wenn eine Gasausstromrate von 1-15 Liter/Minute bereitgestellt wird,
und wobei der Maskenkörper (4) eine im Wesentlichen offene Struktur mit Öffnungen (60) umfasst, die zusammen zwischen 30 und 80% der Gesamtfläche des Maskenkörpers umfassen.

2. Maske gemäß Anspruch 1 in Kombination mit einem Sauerstoffversorgungsschlauch (40), der lösbar an der äußeren Oberfläche (30) des mittleren Abschnitts (16) der Maske befestigt werden kann, um so mit der Sauerstoffversorgungsöffnung (28) in Verbindung zu stehen.

3. Maske gemäß Anspruch 1, wobei die Brückenabschnitte (14) der Maske von einer Oberseite der Maske zu einer Unterseite der Maske in einer umgedrehten "Y"-Form konfiguriert sind, so dass Öffnungen (60) in Richtung der Unterseite und Seiten der Maske bereitgestellt sind zum ungestörten Zugang zu und Sicht auf den Mund eines Patienten und anderen Teilen des Gesichts des Patienten.

4. Maske, wie in Anspruch 1 definiert, wobei der Diffusor (38) so positioniert ist, dass er im Wesentlichen zentriert in der Seite-zu-Seite-Richtung relativ zu dem Gesicht des Patienten ist, wenn die Maske von dem Patienten getragen wird.

5. Maske gemäß Anspruch 1, wobei die Öffnungen (60) zwischen 60 und 75 Prozent der Gesamtfläche des Maskenkörpers (4) umfassen.

6. Maske gemäß Anspruch 1, wobei wenigstens eine der Öffnungen (60) einen Zugang bereitstellt, von außerhalb der Maske zu dem Mund oder Nasenlöchern des Patienten, wenn die Maske von dem Patienten getragen wird.

7. Maske gemäß irgendeinem der Ansprüche 1 bis 6, wobei der Diffusor (38) eine maximale Breite von etwa 40 mm und eine maximale Höhe von etwa 80 mm aufweist.

8. Maske gemäß irgendeinem der Ansprüche 1 bis 7, wobei die Leitplatte (52) einen Pfosten (50) und einen Kopf (56), der eine konische Lippe (54) mit einer geringelten Rückseite umfasst, wobei die Unterseite der Lippe in einer Linie mit Sauerstoff ist, der von der Öffnung (28) vorbeiströmt.

9. Maske gemäß irgendeinem der Ansprüche 1 bis 7, wobei die Maske weiterhin einen Sauerstoff- oder Kohlenstoffdioxid-Überwachungsschlauch (60) umfasst zur Überwachung von Sauerstoff oder Kohlenstoffdioxid.

10. Maske gemäß Anspruch 9, wobei die Leitplatte (64) einen hohlen Pfosten (66) aufweist, dessen hohle Mitte mit einer Öffnung (68) an der Innenseite der Leitplatte (64) und mit dem Überwachungsschlauch (60) in Verbindung steht.

## Revendications

1. Masque (2) pour la délivrance d'oxygène à un patient, le masque comprenant :
un corps (4) ayant une partie périphérique (6), lors de l'utilisation pour reposer confortablement sur le visage d'un patient, une partie centrale (16), et des parties en pont (14) s'étendant entre la partie centrale et la partie périphérique et d'un seul tenant avec celles-ci, la partie centrale (16) ayant une surface intérieure (32) et une surface extérieure (30), la surface intérieure devant être orientée vers le visage du patient lorsque le masque est en position et adaptée au corps humain de manière à reposer à un emplacement s'étendant par-dessus le nez et la bouche du patient, la surface intérieure de la partie centrale étant dotée d'une paroi (34) circonscrivant une base (36), la paroi et la base étant de configuration globalement concave et circonscrivant une ouverture de délivrance d'oxygène (28) positionnée centralement s'étendant à travers la partie centrale entre la surface intérieure et la surface extérieure, la paroi et la base étant configurées pour faire office d'un diffuseur d'oxygène (38) pour diriger le flux d'oxygène globalement vers le nez et la bouche du patient lorsque le masque est utilisé ;
une fixation (24) pour mettre le masque en prise avec le patient de façon à maintenir un contact entre le visage du patient et ladite partie périphérique lorsque le masque est utilisé ;
des moyens (44) associés à l'ouverture (28) de la partie centrale (16) pour recevoir de manière amovible et fixer en position un tube de délivrance d'oxygène (40) ;
ledit diffuseur (38) étant positionné à l'intérieur du masque ; et
une chicane (52, 64) associée audit diffuseur dans le trajet du flux d'oxygène ;
dans lequel la paroi du diffuseur (34) et la base (36) ont une apparence en forme de coupe, la paroi se terminant au niveau d'un bord à contour périphérique triangulaire ;
dans lequel le diffuseur et la chicane sont avec une forme de manière à générer un panache de flux turbulent de gaz enrichi en oxygène qui entoure le nez de l'utilisateur et de préférence également sa bouche lorsqu'un débit de gaz de 1-15 litres/minute est produit ;
et dans lequel le corps de masque (4) comprend une structure sensiblement ouverte ayant des ouvertures (60) qui comprennent, ensemble, entre 30 et 80% de la superficie totale du corps de masque.

2. Masque selon la revendication 1 en combinaison avec un tube de délivrance d'oxygène (40) pouvant être fixé de manière amovible sur la surface extérieure (30) de la partie centrale (16) du masque de manière à communiquer avec l'ouverture de délivrance d'oxygène (28).

3. Masque selon la revendication 1, dans lequel les parties en pont (14) du masque à partir d'un haut du masque jusqu'à un bas du masque sont configurées en forme de « Y » inversé de manière à ce que des ouvertures (60) soient fournies vers le bas et les côtés du masque pour un accès non obstrué à la bouche d'un patient et d'autres parties du visage du patient, et pour voir celles-ci.

4. Masque tel que défini par la revendication 1, dans lequel ledit diffuseur (38) est positionné de manière à être sensiblement centré dans la direction d'un côté à l'autre par rapport audit visage du patient lorsque le masque est porté par le patient.

5. Masque tel que défini par la revendication 1, dans lequel lesdites ouvertures (60) comprennent entre 60 et 75 pour cent de la superficie totale dudit corps de masque (4).

6. Masque tel que défini par la revendication 1, dans lequel au moins l'une desdites ouvertures (60) fournit un accès depuis l'extérieur du masque à la bouche ou aux narines du patient lorsque le masque est porté par un patient.

7. Masque selon l'une quelconque des revendications 1 à 6, dans lequel le diffuseur (38) a une largeur maximale de près de 40 mm et une hauteur maximale de près de 80 mm.

8. Masque selon l'une quelconque des revendications 1 à 7, dans lequel ladite chicane (52) comprend une tige (50) et une tête (56) ayant une lèvre conique (54) recourbée vers l'arrière, le dessous de la lèvre étant en ligne avec l'oxygène passant à partir de l'ouverture (28).

9. Masque selon l'une quelconque des revendications 1 à 7, dans lequel ledit masque comprend en outre un tube de surveillance d'oxygène ou de dioxyde de carbone (60) pour la surveillance de l'oxygène ou du dioxyde de carbone.

10. Masque selon la revendication 9, dans lequel ladite chicane (64) a une tige creuse (66) dont le centre creux communique avec une ouverture (68) sur l'intérieur de la chicane (64) et avec le tube de surveillance (60).
